(19) Europäisches Patentamt
European Patent Office
Office européen des brevets

(11) EP 0 558 369 B1

(12) FASCICULE DE BREVET EUROPEEN

(45) Date de publication et mention de la délivrance du brevet: **28.08.1996 Bulletin 1996/35**

(51) Int. Cl.$^6$: **C07C 213/00**

(21) Numéro de dépôt: **93400358.3**

(22) Date de dépôt: **12.02.1993**

# (54) Procédé de préparation de p-aminophénols

Verfahren zur Herstellung von P-Aminophenolen

Process for the preparation of p-aminophenols

(84) Etats contractants désignés:
**DE FR GB**

(30) Priorité: **28.02.1992 FR 9202346**

(43) Date de publication de la demande:
**01.09.1993 Bulletin 1993/35**

(73) Titulaire: **RHONE-POULENC CHIMIE
92408 Courbevoie Cédex (FR)**

(72) Inventeurs:
- **Gubelmann, Michel M.
F-75016 Paris (FR)**
- **Maliverney, Christian M.
F-69007 Lyon (FR)**

(74) Mandataire: **Dutruc-Rosset, Marie-Claude et al
RHONE-POULENC CHIMIE,
Direction de la Propriété Industrielle,
25, Quai Paul Doumer
92408 Courbevoie Cédex (FR)**

(56) Documents cités:
**US-A- 4 176 138**
**US-A- 4 571 437**
**US-A- 4 885 389**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 558 369 B1

## Description

La présente invention a pour objet un procédé de préparation de p-aminophénols à partir des nitrobenzènes correspondants.

L'invention vise la préparation de composés p-aminophénoliques susceptibles de porter des substituants sur le cycle aromatique.

L'invention concerne également la préparation de composés p-aminophénoliques associés aux composés p-aminophénoliques résultant d'une N-acylation.

L'invention a trait plus particulièrement la préparation du p-aminophénol qui est un composé important d'un point de vue économique, puisqu'il est utilisé comme produit intermédiaire pour la fabrication du N-acétyl p-aminophénol, dénommé de manière simplifiée "APAP".

Il est connu de préparer les p-aminophénols à partir de phénylhydroxylamines selon le réarrangement de Bamberger [E. BAMBERGER - Ber. 27, 1347, 1548 (1894)].

Les phénylhydroxylamines sont préparées par réduction ménagée du nitrobenzène.

Une voie d'accès directe au p-aminophénol consiste à effectuer la réduction catalytique du nitrobenzène. Cette réaction a lieu en milieu aqueux, en présence d'un acide fort, en particulier l'acide sulfurique et d'un catalyseur d'hydrogénation, généralement le platine déposé sur un support.

La production de p-aminophénol peut s'accompagner de la formation de sous-produits tels que l'aniline, l'amino-4 hydroxy-4' diphénylamine, l'amino-4 hydroxy-4' diphényl-éther, la dihydroxy-4,4' diphénylamine, le diamino-4,4' diphényléther.

Afin de imiter la formation des sous-produits, on a préconisé d'ajouter des additifs qui sont, soit des poisons de catalyseurs tels que des composés soufrés (US-A-4 571 437), soit des tensio-actifs (US-A-4 176 138).

Les procédés décrits dans les documents précités nécessitent donc la mise en oeuvre d'additifs supplémentaires.

Par ailleurs, on a décrit dans US-A- 4 885 389 un procédé de préparation de p-aminophénol qui consiste à hydrogéner catalytiquement le nitrobenzène dans un milieu aqueux acide comprenant une petite quantité d'un acide organique.

Un objectif de la présente invention est donc de disposer d'un procédé permettant de minimiser les sous-produits, tout en ne faisant pas appel à des additifs supplémentaires.

Pour pallier les inconvénients précités, la présente invention propose un nouveau procédé de préparation de p-aminophénols.

Il a maintenant été trouvé et c'est ce qui constitue l'objet de la présente invention, un procédé de préparation de p-aminophénol éventuellement substitué, associé éventuellement au composé p-aminophénolique résultant de sa N-acylation, à partir du nitrobenzène correspondant caractérisé par le fait qu'il consiste à hydrogéner le nitrobenzène correspondant, en solution dans un acide monocarboxylique aliphatique saturé et en présence d'une quantité efficace d'un acide protonique.

Le procédé de l'invention s'applique à tout composé benzénique porteur d'un groupe $NO_2$ dont la position en para du groupe $NO_2$ est libre.

Le procédé de l'invention repose sur le fait que l'acide monocarboxylique aliphatique saturé mis en oeuvre joue à la fois le rôle de solvant réactionnel et d'agent nucléophile impliqué dans la transposition de Bamberger, c'est-à-dire la transformation du produit intermédiaire, à savoir un composé benzénique porteur d'une fonction hydroxylamine -NH-OH en un composé benzénique porteur d'un groupe amino et en para, d'un groupe acyloxy. Ce dernier conduit après hydrolyse à un composé benzénique porteur d'un groupe amino et en para, d'un groupe hydroxyle, ou par transacylation à un composé p-aminophénolique N-acylé.

Dans le cas de la préparation du p-aminophénol qui est le composé visé préférentiellement selon l'invention, l'acide acétique permet la transformation de la phénylhydroxylamine en p-acétoxyaniline qui conduit, par hydrolyse au p-aminophénol ou par transacétylation au N-acétyl p-aminophénol.

La portée de la présente invention ne peut en aucun cas être limitée à cette interprétation.

Conformément au procédé de l'invention, on conduit la réaction dans un acide monocarboxylique aliphatique saturé.

On peut mettre en oeuvre selon l'invention, tout acide monocarboxylique aliphatique saturé liquide dans les conditions réactionnelles, de préférence, liquide à la température ambiante. Par température ambiante, on entend généralement une température comprise entre 18 et 25 °C.

Afin de déterminer si un acide monocarboxylique aliphatique saturé convient à la présente invention, on peut se reporter à la littérature, notamment au HANDBOOK of CHEMISTRY and PHYSICS.

Comme exemples d'acides monocarboxylique aliphatiques saturés convenant bien à la présente invention, on peut citer, entre autres, l'acide acétique, l'acide propionique, l'acide butyrique, l'acide isobutyrique, l'acide pentanoïque, l'acide méthyl-2 butanoïque.

Parmi tous les acides monocarboxylique aliphatiques saturés, l'acide acétique est choisi préférentiellement.

Comme mentionné précédemment, l'invention s'applique à tout composé benzénique, porteur d'un groupe $NO_2$ dont la position en para du groupe $NO_2$ est libre.

Parmi les composés benzéniques nitrés, l'invention s'applique tout particulièrement à ceux qui répondent à la formule générale (I) :

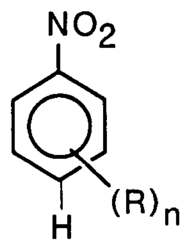

(I)

dans ladite formule (I) :

- R représente un radical alkyle ou alkoxy ayant de 1 à 20 atomes de carbone, de préférence de 1 à 6 atomes de carbone ; un radical alkyle perfluoré ayant de 1 à 4 atomes de carbone ; un atome d'halogène, de préférence le chlore, le brome ou le fluor,
- n est un nombre égal à 0, 1, 2,3 ou 4, de préférence égal à 0,1 ou 2.

La présente invention n'exclut pas la présence d'autres substituants sur le cycle aromatique dans la mesure où ils n'interfèrent pas avec les réactions du procédé de l'invention. En particulier, il est possible qu'il y ait présence de groupes fonctionnels ou d'atomes d'halogène sur les chaînes alkyle portées par le noyau benzénique ou que ces chaînes alkyle soient interrompues par un hétéroatome tel que l'oxygène, l'azote ou le soufre.

Dans l'exposé qui suit de la présente invention, on utilisera le terme de "nitrobenzène" de manière générique. Il désignera aussi bien le nitrobenzène que tous les composés benzéniques nitrés, en particulier ceux répondant à la formule (I).

Comme exemples de nitrobenzènes de formule (I), on peut citer notamment :

- le nitrobenzène,
- l'o-nitrotoluène,
- le m-nitrotoluène,
- le diméthyl-2,6 nitrobenzène,
- l'o-chloronitrobenzène,
- le m-chloronitrobenzène
- l'o-trifluorométhylnitrobenzène
- le m-trifluorométhylnitrobenzène.

Le réactif de départ intervenant dans le procédé de l'invention est le nitrobenzène. On peut faire appel au produit disponible sur le marché qui présente, de préférence, une pureté supérieure à 99 %. Il est souhaitable de mettre en oeuvre un réactif contenant moins de 1 % d'eau.

L'acide monocarboxylique aliphatique saturé étant utilisé à la fois comme réactif et comme solvant réactionnel, sa quantité mise en oeuvre peut varier très largement.

Ainsi, la quantité d'acide monocarboxylique aliphatique saturé engagée est d'au moins 1 mole par mole de nitrobenzène. On peut en utiliser un excès notable de sorte que la quantité d'acide monocarboxylique aliphatique saturé peut atteindre jusqu'à 500 moles par mole de nitrobenzène. D'une manière préférée, on met en jeu de 100 à 300 moles d'acide monocarboxylique aliphatique saturé par mole de nitrobenzène.

Un mode préféré de réalisation de l'invention consiste à faire appel à l'acide acétique. On met en oeuvre préférentiellement dans le procédé de l'invention l'acide acétique glacial c'est-à-dire un acide acétique ayant une pureté supérieure à 99,8 %.

A côté de l'acide monocarboxylique aliphatique saturé, on met en oeuvre selon l'invention, un acide protonique ayant un pKa inférieur ou égal à 4,00. Encore plus préférentiellement, on fait appel à un acide protonique ayant un pKa inférieur ou égal à 3,00.

Le pKa est défini comme la constante de dissociation ionique du couple acide/base, lorsque l'eau est utilisée comme solvant.

Pour le choix d'un acide ayant un pKa tel que défini par l'invention, on peut se reporter, entre autres, au HANDBOOK OF CHEMISTRY AND PHYSICS, 66ème édition, p. D-161 ET D-162.

Dans le choix de l'acide protonique mis en oeuvre dans le procédé de l'invention, il y a lieu de veiller que celui-ci présente une nucléophilie inférieure à celle de l'acide monocarboxylique aliphatique saturé, c'est-à-dire qu'il ne réagisse pas sur le noyau aromatique.

Comme exemples d'acides protoniques convenant à l'invention, on peut citer plus particulièrement les oxacides minéraux halogénés ou non, tels que l'acide sulfurique, l'acide chlorosulfonique, l'acide fluorosulfonique ; les acides phosphoriques tels que l'acide phosphorique, l'acide (éthyl-2 hexyl) phosphorique, l'acide (octylphényl) phosphorique; les acides phosphoniques tels que, par exemple, l'acide (éthyl-2 hexyl) (éthyl-2 hexyl) phosphonique ; les acides carboxyliques perhalogénés ou non tels que l'acide formique, l'acide citrique, l'acide trichloroacétique, l'acide trifluoroacétique. Les acides sulfoniques halogénés ou non sont également bien adaptés à la présente invention. Parmi ceux-ci, on peut citer l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques, les acides naphtalènedisulfoniques, les acides camphorsulfoniques.

Parmi ces acides, on utilisera de préférence l'acide sulfurique et les acides sulfoniques.

La quantité d'acide protonique tel que précité, exprimée par le rapport du nombre d'équivalents de protons au nombre de moles de nitrobenzène peut varier dans de larges limites. Ainsi, le rapport $H^+$/nitrobenzène peut varier entre 0,1 et 5,0, et de préférence entre 0,5 et 3,0.

On fait appel, de préférence, aux acides concentrés du commerce.

L'hydrogénation du nitrobenzène est effectuée de manière classique c'est-à-dire par mise au contact du nitrobenzène avec l'hydrogène, en présence d'un catalyseur d'hydrogénation.

Le catalyseur peut être au moins un métal noble choisi parmi le platine et le palladium. Ledit métal peut être apporté sous une forme finement divisée ou bien déposé sur un support. Comme exemples de supports, on peut mentionner le charbon, le noir d'acétylène, la silice, l'alumine, la zircone, l'oxyde de chrome, la bentonite etc...

Le métal peut être déposé sur le support sous forme métallique ou bien sous la forme d'un composé qui sera réduit en métal en présence d'hydrogène. On peut entre autres mettre en oeuvre un oxyde de platine et/ou de palladium.

Parmi les catalyseurs précités, le catalyseur préféré est le platine déposé sur charbon.

De préférence, le platine et/ou le palladium est déposé sur un support. Généralement, il est déposé à raison de 0,5 % à 5 % du poids du catalyseur.

Le catalyseur peut être mis en oeuvre sous forme d'une poudre, de pellets ou bien de granulés.

La quantité de catalyseur d'hydrogénation mis en oeuvre, exprimée en atome-gramme de métal noble par mole de nitrobenzène peut varier, par exemple, entre $1.10^{-5}$ et $1.10^{-2}$, de préférence entre $5.10^{-5}$ et $1.10^{-3}$.

Le procédé de l'invention est conduit à une température choisie dans une gamme de températures allant de 50°C à 200°C et choisie plus particulièrement entre 80°C et 150°C.

La réaction se déroule sous pression d'hydrogène allant d'une pression légèrement supérieure à la pression atmosphérique jusqu'à une pression de plusieurs centaines de bars. Avantageusement, la pression d'hydrogène varie entre 1 et 20 bars, et plus préférentiellement entre 3 et 10 bars.

La durée de la réaction peut être variable. Elle dépend de la nature de l'agent protonique, de la concentration en nitrobenzène, de la teneur en catalyseur, de la pression et de la température réactionnelle. Elle se situe, préférentiellement, entre 30 minutes et 5 heures.

D'un point de vue pratique, la réaction est aisément réalisée en chargeant dans l'appareillage, tous les réactifs en même temps, à savoir le nitrobenzène, l'acide monocarboxylique aliphatique saturé, l'acide protonique ayant un pKa inférieur ou égal à 4,00 et le catalyseur d'hydrogénation.

Après mise en oeuvre de tous les réactifs, on porte le milieu réactionnel à la température choisie tout en établissant une atmosphère d'hydrogène à la pression souhaitée, le mélange réactionnel étant maintenu sous faible agitation, par exemple, entre 500 et 1000 tours/minute.

Lorsque la température réactionnelle est atteinte, l'agitation est augmentée jusqu'à environ 2000 tours/minute.

On maintient le milieu réactionnel sous agitation, pendant toute la durée de la réaction.

En fin de réaction, on sépare le catalyseur en suspension, et le composé p-aminophénolique obtenu, selon les techniques classiques utilisées dans le domaine considéré.

Le procédé de l'invention conduit à un composé p-aminophénolique obtenu en quantité majoritaire.

Il est possible selon les conditions opératoires, en particulier en faisant appel à à certains acides protoniques par exemple l'acide phosphorique, d'obtenir directement le composé p-aminophénolique N-acylé, à côté du composé p-aminophénolique.

Conformément au procédé de l'invention, on obtient essentiellement un composé p-aminophénolique qui peut servir, entre autres, comme produit intermédiaire pour la préparation d'un composé p-aminophénolique N-acylé.

Tel est le cas lors de la préparation du N-acétyl p-aminophénol qui est obtenu par acétylation du p-aminophénol. L'acétylation effectuée de manière connue, est réalisée de préférence, avec l'anhydride acétique.

Les exemples qui suivent, illustrent l'invention sans toutefois la limiter.

Dans les exemples, les abréviations signifient:

- PAP = p-aminophénol
- APAP = N-acétyl p-aminophénol

$$\text{Conversion} = \frac{\text{nombre de moles de nitrobenzène transformées}}{\text{nombre de moles de nitrobenzène introduites}}$$

$$\text{Rendement} = \frac{\text{nombre de moles de PAP formées}}{\text{nombre de moles de nitrobenzène introduites}}$$

$$\text{Sélectivité} = \frac{\text{nombre de moles de PAP + APAP formées}}{\text{nombre de moles PAP + APAP + aniline + acétanilide formées}}$$

EXEMPLES

On donne ci-après le protocole opératoire qui sera utilisé dans tous les exemples.

Dans un réacteur de 70 $cm^3$ équipé d'une agitation par turbine RUSHTON, d'un dispositif de chauffage, d'un dispositif de régulation de température et d'une arrivée d'hydrogène, on charge les réactifs suivants:

- 2,46 g de nitrobenzène (20 mmol),
- 15 $cm^3$ d'acide acétique glacial,
- 20 mmol d'acide protonique,
- 1,15. $10^{-5}$ atome-gramme de platine déposé sur charbon à raison de 5 % en poids.

On porte le milieu réactionnel à une température de 120°C tout en établissant une atmosphère d'hydrogène de 5 bars, avec une agitation initiale de 1000 tours/minute.

Lorsque la température de 120°C est atteinte, l'agitation est augmentée à 2000 tours/minute.

La durée de la réaction est indiquée dans le tableau récapitulatif joint.

En fin de réaction, on dose les réactifs restants et les produits formés par chromatographie liquide haute performance.

Exemples 1 à 4 :

On effectue quatre essais en mettant en oeuvre différents acides protoniques.

Les résultats obtenus sont donnés dans la tableau I.

Tableau I

| N° ex. | Acide fort | Durée en heures | Conversion $PhNO_2$ (%) | Rendement (%) | | | | Sélectivité (%) |
|---|---|---|---|---|---|---|---|---|
| | | | | PAP | APAP | $PhNH_2$ | PhNHAc | |
| 1 | $H_3PO_4$ | 6h | 100 | 7,4 | 10,2 | 12,9 | 56,0 | 21 |
| 2 | $PhSO_3H$ | 4h | 100 | 17,4 | 0,1 | 45,3 | 11,7 | 24 |
| 3 | $H_2SO_4$ | 3h | 45 | 30,2 | 0,1 | 14,2 | 0,2 | 68 |
| 4 | $CF_3SO_3H$ | 1h45 | 100 | 45,3 | 1,9 | 24,4 | 1,7 | 65 |

L'examen du tableau (I) met en évidence que les meilleurs rendements et sélectivités sont obtenus lorsque l'on fait appel à l'acide sulfurique ou à l'acide trifluorométhanesulfonique, comme acide protonique.

## Revendications

**1.** Procédé de préparation de p-aminophénol éventuellement substitué, associé éventuellement au composé p-aminophénolique résultant de sa N-acylation, à partir du nitrobenzène correspondant caractérisé par le fait qu'il consiste à hydrogéner le nitrobenzène correspondant, en solution dans un acide monocarboxylique aliphatique saturé et en présence d'une quantité efficace d'un acide protonique.

2. Procédé selon la revendication 1 caractérisé par le fait que le composé benzénique nitré répond à la formule (I) suivante :

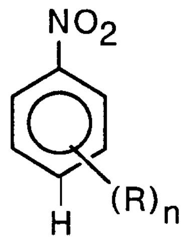

(I)

dans ladite formule (I) :

- R représente un radical alkyle ou alkoxy ayant de 1 à 20 atomes de carbone, de préférence de 1 à 6 atomes de carbone ; un radical alkyle perfluoré ayant de 1 à 4 atomes de carbone ; un atome d'halogène, de préférence le chlore, le brome ou le fluor,
- n est un nombre égal à 0, 1, 2,3 ou 4, de préférence égal à 0,1 ou 2.

3. Procédé selon l'une des revendications 1 et 2 caractérisé par le fait que le composé benzénique nitré est choisi parmi :

- le nitrobenzène,
- l'o-nitrotoluène,
- le m-nitrotoluène,
- le diméthyl-2,6 nitrobenzène,
- l'o-chloronitrobenzène,
- le m-chloronitrobenzène
- l'o-trifluorométhylnitrobenzène
- le m-trifluorométhylnitrobenzène.

4. Procédé selon l'une des revendications 1 à 3 caractérisé par le fait que l'acide monocarboxylique aliphatique saturé mis en oeuvre est l'acide acétique, l'acide propionique, l'acide butyrique, l'acide isobutyrique, l'acide pentanoïque, l'acide méthyl-2 butanoïque.

5. Procédé selon l'une des revendications 1 à 4 caractérisé par le fait que l'acide monocarboxylique aliphatique saturé mis en oeuvre est l'acide acétique glacial.

6. Procédé selon l'une des revendications 1 à 5 caractérisé par le fait que la quantité d'acide monocarboxylique aliphatique saturé engagée est d'au moins 1 mole par mole de nitrobenzène et peut atteindre jusqu'à 500 moles par mole de nitrobenzène.

7. Procédé selon la revendication 6 caractérisé par le fait que la quantité d'acide monocarboxylique aliphatique saturé engagée représente de 100 à 300 moles d'acide monocarboxylique aliphatique saturé par mole de nitrobenzène.

8. Procédé selon l'une des revendications 1 à 7 caractérisé par le fait que l'acide protonique est un acide protonique ayant un pKa inférieur ou égal à 4,00, de préférence, inférieur ou égal à 3,00.

9. Procédé selon l'une des revendications 1 à 8 caractérisé par le fait que l'acide protonique est choisi parmi : les oxacides minéraux halogénés ou non ; les acides phosphoriques ; les acides phosphoniques ; les acides carboxyliques perhalogénés ou non ; les acides sulfoniques halogénés ou non.

10. Procédé selon l'une des revendications 1 à 9 caractérisé par le fait que l'acide protonique est choisi parmi : l'acide sulfurique, l'acide chlorosulfonique, l'acide fluorosulfonique, l'acide phosphorique, l'acide (éthyl-2 hexyl) phosphorique, l'acide (octylphényl) phosphorique, l'acide (éthyl-2 hexyl) (éthyl-2 hexyl) phosphonique, l'acide formique, l'acide citrique, l'acide trichloroacétique, l'acide trifluoroacétique, l'acide fluorosulfonique, l'acide chlorosulfonique, l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques, les acides naphtalènedisulfoniques, les acides camphorsulfoniques.

**11.** Procédé selon l'une des revendications 1 à 10 caractérisé par le fait que l'acide protonique est choisi parmi l'acide sulfurique et les acides sulfoniques.

**12.** Procédé selon l'une des revendications 1 à 11 caractérisé par le fait que la quantité d'acide protonique exprimée par le rapport du nombre d'équivalents de protons au nombre de moles de nitrobenzène varie entre 0,1 et 5,0, et de préférence entre 0,5 et 3,0.

**13.** Procédé selon l'une des revendications 1 à 12 caractérisé par le fait que le nitrobenzène est mis au contact de l'hydrogène, en présence d'un catalyseur d'hydrogénation choisi parmi le platine et le palladium, éventuellement déposé sur un support.

**14.** Procédé selon l'une des revendications 1 à 13 caractérisé par le fait que la quantité de catalyseur d'hydrogénation mis en oeuvre, exprimée en atome-gramme de métal noble par mole de nitrobenzène varie entre $1.10^{-5}$ et $1.10^{-2}$, de préférence entre $5.10^{-5}$ et $1.10^{-3}$.

**15.** Procédé selon l'une des revendications 1 à 14 caractérisé par le fait que la température réactionnelle est choisie dans une gamme de températures allant de 50°C à 200°C et plus particulièrement entre 80°C et 150°C.

**16.** Procédé selon l'une des revendications 1 à 15 caractérisé par le fait que la réaction se déroule sous pression d'hydrogène comprise entre 1 et 20 bars, et, de préférence entre 3 et 10 bars.

**17.** Procédé de préparation de p-aminophénol, à partir du nitrobenzène caractérisé par le fait qu'il consiste à hydrogéner selon l'une des revendications 1 à 16, le nitrobenzène, en solution dans un acide monocarboxylique aliphatique saturé et en présence d'une quantité efficace d'un acide protonique, puis à effectuer l'acétylation du p-aminophénol obtenu.

**18.** Procédé selon la revendication 17 caractérisé par le fait que l'acétylation est effectuée à l'aide d'anhydride acétique.

## Claims

**1.** A process for the preparation of optionally substituted p-amino-phenol, possibly associated with the p-amino-phenolic compound resulting from its N-acylation, from the corresponding nitrobenzene, characterised in that it consists in hydrogenating the corresponding nitrobenzene in solution in a saturated aliphatic monocarboxylic acid and in the presence of an effective quantity of a protonic acid.

**2.** A process according to Claim 1, characterised in that the nitrated benzene compound corresponds to the following formula (I):

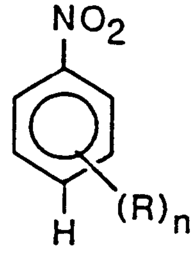

(I)

in which formula (I):

- R represents an alkyl or alkoxy radical with 1 to 20 carbon atoms, preferably 1 to 6 carbon atoms; a perfluoroalkyl radical with 1 to 4 carbon atoms; a halogen atom, preferably chlorine, bromine or fluorine,
- n is a number equal to 0, 1, 2, 3 or 4, preferably equal to 0, 1 or 2.

**3.** A process according to Claim 1 or Claim 2, characterised in that the nitrated benzene compound is selected from:

- nitrobenzene,
- o-nitrotoluene,
- m-nitrotoluene,
- 2,6-dimethyl nitrobenzene,
- o-chloronitrobenzene,
- m-chloronitrobenzene
- o-trifluoromethyl nitrobenzene
- m-trifluoromethyl nitrobenzene.

**4.** A process according to one of Claims 1 to 3, characterised in that the saturated aliphatic monocarboxylic acid used is acetic acid, propionic acid, butyric acid, isobutyric acid, pentanoic acid, 2-methyl butanoic acid.

**5.** A process according to one of Claims 1 to 4, characterised in that the saturated aliphatic monocarboxylic acid used is glacial acetic acid.

**6.** A process according to one of Claims 1 to 5, characterised in that the amount of saturated aliphatic monocarboxylic acid used is at least 1 mole per mole of nitrobenzene and can be up to 500 moles per mole of nitrobenzene.

**7.** A process according to Claim 6, characterised in that the amount of saturated aliphatic monocarboxylic acid used represents between 100 and 300 moles of saturated aliphatic monocarboxylic acid per mole of nitrobenzene.

**8.** A process according to one of Claims 1 to 7, characterised in that the protonic acid is a protonic acid with a pKa which is less than or equal to 4.00, preferably less than or equal to 3.00.

**9.** A process according to one of Claims 1 to 8, characterised in that the protonic acid is selected from: halogenated or non-halogenated mineral oxacids; phosphoric acids; phosphonic acids; perhalogenated or non-perhalogenated carboxylic acids; halogenated or non-halogenated sulphonic acids.

**10.** A process according to one of Claims 1 to 9, characterised in that the protonic acid is selected from: sulphuric acid, chlorosulphonic acid, fluorosulphonic acid, phosphoric acid, (2-ethyl hexyl) phosphoric acid, (octylphenyl) phosphoric acid, (2-ethyl hexyl) (2-ethyl hexyl) phosphonic acid, formic acid, citric acid, trichloroacetic acid, trifluoroacetic acid, fluorosulphonic acid, chlorosulphonic acid, trifluoromethane sulphonic acid, methane sulphonic acid, ethane sulphonic acid, ethane disulphonic acid, benzene sulphonic acid, benzene disulphonic acids, toluene sulphonic acids, naphthalene sulphonic acids, naphthalene disulphonic acids, camphor sulphonic acids.

**11.** A process according to one of Claims 1 to 10, characterised in that the protonic acid is selected from sulphuric acid and sulphonic acids.

**12.** A process according to one of Claims 1 to 11, characterised in that the amount of protonic acid, expressed by the ratio of the number of equivalents of protons to the number of moles of nitrobenzene varies between 0.1 and 5.0, and preferably between 0.5 and 3.0.

**13.** A process according to one of Claims 1 to 12, characterised in that the nitrobenzene is contacted with the hydrogen, in the presence of a hydrogenation catalyst selected from platinum and palladium, possibly deposited on a support.

**14.** A process according to one of Claims 1 to 13, characterised in that the amount of hydrogenation catalyst used, expressed in gramme atom of precious metal per mole of nitrobenzene varies between $1 \times 10^{-5}$ and $1 \times 10^{-2}$, preferably between $5 \times 10^{-5}$ and $1 \times 10^{-3}$.

**15.** A process according to one of Claims 1 to 14, characterised in that the reaction temperature is selected within a temperature range between 50°C and 200°C, and more particularly between 80°C and 150°C.

**16.** A process according to one of Claims 1 to 15, characterised in that the reaction takes place under hydrogen pressure between 1 and 20 bars, preferably between 3 and 10 bars.

**17.** A process for the preparation of p-aminophenol, from nitrobenzene, characterised in that it consists in the hydrogenation, according to one of Claims 1 to 16, of the nitrobenzene in solution in a saturated aliphatic monocarboxylic acid and in the presence of an effective amount of a protonic acid, followed by the effecting of acetylation of the p-aminophenol obtained.

**18.** A process according to Claim 17, characterised in that the acetylation is carried out by means of acetic anhydride.

**Patentansprüche**

**1.** Verfahren zur Herstellung von gegebenenfalls substituiertem p-Aminophenol sowie gegebenenfalls von der aus dessen N-Acylierung resultierenden p-Aminophenolverbindung, ausgehend von dem entsprechenden Nitrobenzol, dadurch gekennzeichnet, daß man das in einer Lösung aus einer gesättigten aliphatischen Monocarbonsäure befindliche entsprechende Nitrobenzol in Gegenwart einer wirksamen Menge einer Protonensäure hydrogeniert.

**2.** Verfahren nach Anspruch 1, dadurch gekennzeichnet, daß die nitrierte Benzolverbindung der folgenden Formel (I) entspricht:

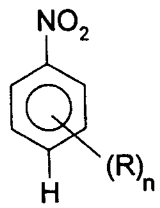

(I)

wobei in dieser Formel (I):

- R einen Alkyl- oder Alkoxyrest mit 1 bis 20 Kohlenstoffatomen, vorzugsweise mit 1 bis 6 Kohlenstoffatomen; einen perfluorierten Alkylrest mit 1 bis 4 Kohlenstoffatomen; ein Halogenatom, vorzugsweise Chlor, Brom oder Fluor, darstellt,
- n eine Zahl von 0, 1, 2, 3 oder 4, vorzugsweise von 0,1 oder 2, darstellt.

**3.** Verfahren nach Anspruch 1 oder 2, dadurch gekennzeichnet, daß die nitrierte Benzolverbindung ausgewählt ist aus:

- Nitrobenzol,
- o-Nitrotoluol,
- m-Nitrotoluol,
- 2,6-Dimethylnitrobenzol,
- o-Chlornitrobenzol,
- m-Chlornitrobenzol,
- o-Trifluormethylnitrobenzol,
- m-Trifluormethylnitrobenzol.

**4.** Verfahren nach einem der Ansprüche 1 bis 3, dadurch gekennzeichnet, daß die verwendete gesättigte aliphatische Monocarbonsäure Essigsäure, Propionsäure, Buttersäure, Isobuttersäure, Pentansäure der 2-Methylbutansäure ist.

**5.** Verfahren nach einem der Ansprüche 1 bis 4, dadurch gekennzeichnet, daß die verwendete gesättigte aliphatische Monocarbonsäure Eisessig ist.

**6.** Verfahren nach einem der Ansprüche 1 bis 5, dadurch gekennzeichnet, daß die Menge der eingesetzten gesättigten aliphatischen Monocarbonsäure mindestens 1 mol pro mol Nitrobenzol beträgt und bis zu 500 mol pro mol Nitrobenzol betragen kann.

7. Verfahren nach Anspruch 6, dadurch gekennzeichnet, daß die Menge an eingesetzter gesättigter aliphatischer Monocarbonsäure 100 bis 300 mol gesättigter aliphatischer Monocarbonsäure pro mol Nitrobenzol beträgt.

8. Verfahren nach einem der Ansprüche 1 bis 7, dadurch gekennzeichnet, daß die Protonensäure eine Protonensäure mit einem $pK_a$-Wert von kleiner oder gleich 4,00, vorzugsweise kleiner oder gleich 3,00, ist.

9. Verfahren nach einem der Ansprüche 1 bis 8, dadurch gekennzeichnet, daß die Protonensäure ausgewählt ist aus: halogenierten oder nichthalogenierten anorganischen Sauerstoffsäuren; Phosphorsäuren, Phosphonsäuren, perhalogenierten oder nichtperhalogenierten Carbonsäuren; halogenierten oder nichthalogenierten Sulfonsäuren.

10. Verfahren nach einem der Ansprüche 1 bis 9, dadurch gekennzeichnet, daß die Protonensäure ausgewählt ist aus: Schwefelsäure, Chlorsulfonsäure, Fluorsulfonsäure, Phosphorsäure, 2-Ethylhexylphosphorsäure, Octylphenylphosphorsäure, 2-Ethylhexylphosphonsäure, Ameisensäure, Zitronensäure, Trichloressigsäure, Trifluoressigsäure, Fluorsulfonsäure, Chlorsulfonsäure, Trifluormethansulfonsäure, Methansulfonsäure, Ethansulfonsäure, Ethandisulfonsäure, Benzolsulfonsäure, Benzoldisulfonsäuren, Toluolsulfonsäuren, Naphthalinsulfonsäuren, Naphthalindisulfonsäuren und Camphersulfonsäuren.

11. Verfahren nach einem der Ansprüche 1 bis 10, dadurch gekennzeichnet, daß die Protonensäure ausgewählt ist aus Schwefelsäure und Sulfonsäuren.

12. Verfahren nach einem der Ansprüche 1 bis 11, dadurch gekennzeichnet, daß die Menge an Protonensäure, bezogen auf das Verhältnis der Zahl der Protonenäquivalenten zu der Molanzahl von Nitrobenzol 0,1 bis 5,0, vorzugsweise 0,5 bis 3,0, beträgt.

13. Verfahren nach einem der Ansprüche 1 bis 12, dadurch gekennzeichnet, daß das Nitrobenzol in Gegenwart eines Hydrogenierungskatalysators mit dem Wasserstoff in Kontakt gebracht wird, der ausgewählt ist aus Platin und Palladium, und gegebenenfalls auf ein Trägermaterial aufgebracht ist.

14. Verfahren nach einem der Ansprüche 1 bis 13, dadurch gekennzeichnet, daß die Menge an verwendetem Hydrogenierungskatalysator, bezogen auf die Grammatomanzahl von Edelmetall pro mol Nitrobenzol zwischen $1 \cdot 10^{-5}$ und $1 \cdot 10^{-2}$, vorzugsweise zwischen $5 \cdot 10^{-5}$ und $1 \cdot 10^{-3}$, liegt.

15. Verfahren nach einem der Ansprüche 1 bis 14, dadurch gekennzeichnet, daß die Reaktionstemperatur zwischen 50 und 200 °C, insbesondere zwischen 80 und 150 °C, liegt.

16. Verfahren nach einem der Ansprüche 1 bis 15, dadurch gekennzeichnet, daß die Reaktion unter einem Wasserstoffdruck zwischen 1 und 20 bar, vorzugsweise zwischen 3 und 10 bar, durchgeführt wird.

17. Verfahren zur Herstellung von p-Aminophenol, ausgehend von Nitrobenzol, dadurch gekennzeichnet, daß man Nitrobenzol in einer gesättigten aliphatischen Monocarbonsäurelösung und in Gegenwart einer wirksamen Menge einer Protonensäure nach einem der Ansprüche 1 bis 16 hydrogeniert und dann das erhaltene p-Aminophenol acetyliert.

18. Verfahren nach Anspruch 17, dadurch gekennzeichnet, daß die Acetylierung mit Hilfe von Essigsäureanhydrid durchgeführt wird.